# EUROPÄISCHE PATENTANMELDUNG

(11) **EP 1 790 325 A1**
(43) Veröffentlichungstag der Anmeldung: **30.05.2007**
(21) Anmeldenummer: 06124748.2
(22) Anmeldetag: 24.11.2006
(51) Int. Cl.: A61K 8/02, A61K 8/73, A61K 8/81, A61Q 5/12, A61Q 15/00, A61Q 17/04, A61Q 19/00, A61Q 19/10, A61K 8/891

(54) **Kosmetische Hydrodispersionen**

(30) Priorität: 28.11.2005 DE 102005056498
(71) Anmelder: Beiersdorf AG, 20245 Hamburg (DE)
(72) Erfinder: Bleckmann, Andreas, 22926, Ahrensburg (DE); Riedel, Heidi, 22083, Hamburg (DE)

(57) **Zusammenfassung**

Kosmetische Zubereitung in Form einer Hydrodispersion, dadurch gekennzeichnet, dass sie
i. Wasser,
ii. Natriumcarboxymethylstärke,
iii. ein oder mehrere Hydrokolloide,
iv. ein oder mehrere Silikonöle und
v. bis zu 2 Gew.-% eines oder mehrerer Emulgatoren enthält.

## Beschreibung

Die vorliegende Erfindung betrifft kosmetische Zubereitungen in Form von Hydrodispersionen mit einer besonderen samtigen Textur.

Häufige Erscheinungsformen kosmetischer Zubereitungen sind feindisperse Mehrphasensysteme, in welchen eine oder mehrere Fett- bzw. Ölphasen neben einer bzw. mehreren Wasserphasen vorliegen. Von diesen Systemen sind wiederum die eigentlichen Emulsionen die am weitesten verbreiteten.

Aber auch emulgatorarme bzw. -freie Zubereitungen auf Basis sogenannter Hydrodispersionen sind seit einiger Zeit bekannt. Hydrodispersionen stellen Dispersionen einer flüssigen, halbfesten oder festen inneren (diskontinuierlichen) Lipidphase in einer äußeren wäßrigen (kontinuierlichen) Phase dar. Hydrodispersionen sind - wie auch Emulsionen, die sich durch eine ähnliche Phasenanordnung auszeichnen - metastabile Systeme und daher geneigt, in einen Zustand zweier in sich zusammenhängender diskreter Phasen überzugehen. In einer klassischen O/W-Emulsion verhindert die Wahl eines geeigneten Emulgators die Phasentrennung. Im Gegensatz zur klassischen Emulsionen enthalten Hydrodispersionen aber nur sehr geringe Mengen an Emulgatoren (bis zu 2 Gew.-%) bzw. können sogar gänzlich frei von Emulgatoren sein.

Bei Hydrodispersionen, welche aus einer flüssigen Lipidphase in einer äußeren wässrigen Phase bestehen, wird die Stabilität des Mehrphasensystems üblicherweise dadurch gewährleistet, dass in der wässrigen Phase mit Hilfe eines Gelbildners ein Gelgerüst aufgebaut wird, in welchem die Lipidtröpfchen stabil suspendiert sind.

Obwohl dieser Formulierungstyp gegenüber herkömmlichen Emulsionen den Vorteil der Emulgatorarmut bzw. -freiheit bietet, gibt es andererseits auch einige Punkte, welche der Verbesserung bedürfen. So fühlen sich Hydrodispersionen im Vergleich zu Emulsionen häufig klebrig an. Ein weiterer Nachteil von Hydrodispersionen, insbesondere solcher, die Acrylate als Gelbildner enthalten, ist der unvorteilhafte Abrieb, wenn die auf die Haut aufgetragene Hydrogelschicht leicht und in unschöner Weise ("Röllchenbildung") abgetragen wird.

Nachteil der Hydrodispersionen des Standes der Technik ist ferner, dass diese bei einem höheren Gehalt an Lipiden (größer 4 Gew.-%) zur Phasentrennung neigen, die insbesondere bei höheren Temperaturen (≥ 40 °C) auftreten kann.

Unter Kosmetik kann man alle Maßnahmen zusammenfassen, die aus ästhetischen Gründen Veränderungen an Haut und Haaren vornehmen oder zur Körperreinigung angewendet werden. Kosmetik bedeutet also, das Körperäußere zu pflegen, zu verbessern und zu verschönern, um auf sichtbare, fühlbare und riechbare Weise sowohl den Mitmenschen als auch sich selbst zu gefallen. Schon vor Jahrtausenden wurde Kosmetik vom Menschen zu diesem Zweck angewandt. Man färbte Lippen und Gesicht, salbte sich mit wertvollen Ölen und badete in duftendem Wasser.

Während die Reinigungswirkung eines Shampoos, die Kämmbarkeitsverbesserung einer Haarspülung oder die Kräuselung des Haares durch eine Dauerwelle einfach und objektiv feststellbar sind, sind andere Wirkungen und Eigenschaften kosmetischer Produkte praktisch nicht messbar oder nur sehr individuell spürbar. Hierzu zählen zum Beispiel ein bestimmtes (belebendes, weiches, geschmeidiges, glattes etc.) Hautgefühl oder die Weichheit und Geschmeidigkeit der Haut nach der Anwendung eines Kosmetikums sowie die Fülle und Sprungkraft der Haare und dergleichen mehr. Ferner unterliegt die Erwartung der Verbraucher auch nebengeordneten Eigenschaften des Produktes. Hier sind insbesondere der Duft und die Farbe bzw. das Aussehen des Kosmetikums sowie seine Verpackung, der Preis, der Hersteller und die Werbeaussage zu nennen.

Eine für den Verbraucher sehr wesentliche, dabei aber nur schwer quantitativ messbare Eigenschaft kosmetischer Produkte ist ihre Textur. Unter dem Begriff "Textur" werden diejenigen Eigenschaften eines Kosmetikums verstanden, die auf den Gefügebau der Zubereitung zurückgehen, durch Tast- und Berührungssinne empfunden und ggf. in mechanischen oder rheologischen Fließeigenschaften ausgedrückt werden können. Die Textur kann insbesondere mittels Sensorik getestet werden. Die gegebenenfalls mit Hilfe von Zusatzstoffen beeinflussbare Textur kosmetischer Produkte ist für den Verbraucher von nahezu gleicher Bedeutung wie deren objektiv feststellbaren Wirkungen.

Mit dem Begriff "Sensorik" wird die wissenschaftliche Disziplin bezeichnet, die sich mit der Bewertung von kosmetischen Zubereitungen auf Grund von Sinneseindrücken befasst. Die sensorische Beurteilung eines Kosmetikums erfolgt anhand der visuellen, olfaktorischen und haptischen Eindrücke.
- *Visuelle Eindrücke:* alle mit dem Auge wahrnehmbaren Merkmale (Farbe, Form, Struktur).
- *Olfaktorische Eindrücke:* alle beim Einziehen von Luft durch die Nase wahrnehmbaren Geruchseindrücke, die häufig in Anfangsgeruch (Kopfnote), Hauptgeruch (Mittelnote, Körper) und Nachgeruch (Ausklang) differenziert werden können. Auch die erst bei der Anwendung freigesetzten flüchtigen Stoffe tragen zum olfaktorischen Eindruck bei.
- *Haptische Eindrücke:* alle Empfindungen des Tastsinns, die vornehmlich Gefüge und Konsistenz des Produktes betreffen.

Die sensorische Analyse macht von der Möglichkeit Gebrauch, den sensorischen Gesamteindruck eines Produktes integral zu erfassen. Nachteile der sensorische Analyse sind die Subjektivität des Eindrucks, eine leichte Beeinflussbarkeit der Prüfpersonen und die dadurch bedingte starke Streuung der Ergebnisse. Diesen Schwächen begegnet man heute durch den Einsatz von Gruppen geschulter Prüfpersonen, gegenseitige Abschirmung der Prüfer sowie statistische Auswertung der meist zahlreichen Analysendaten.

Die am häufigsten in der Forschung und Entwicklung genutzte Methode der sensorischen Analyse ist die Unterschiedsprüfung. Die Aufgabe beschränkt sich hierbei üblicherweise auf die Erkennung eines von mehreren Proben oder von einer Kontrollprobe abweichenden Musters. Während bei Unterschiedsprüfungen innerhalb eines Tests nur zwei Proben miteinander verglichen werden, ist bei der Rangordnungsprüfung eine Reihenfolge von drei und mehr Proben festzulegen und zwar in der Regel nach Intensität, Qualität, Beliebtheit oder Ähnlichkeit mit einer Vergleichsprobe. Diese (einfache) Methode eignet sich z. B. für eine Vorauswahl von Proben in der Produkt-Optimierung und wird auch häufig in der Marktforschung eingesetzt.

Eine Aufgabe der vorliegenden Erfindung war es, emulgatorarme bzw. emulgatorfreie Zubereitungen zu finden, welche neben den für Kosmetika üblichen Kriterien wie Verträglichkeit, Lagerstabilität und dergleichen auch für den Verbraucher wesentliche, bisher nicht gekannte kosmetische Leistungen bieten. Insbesondere sollten die gesuchten Zubereitungen die Nachteile des Standes der Technik nicht aufweisen, d. h. sie sollten gehaltvoll und pflegend sein und dabei gleichzeitig gute sensorische Eigenschaften aufweisen. Demensprechend sollten die Zubereitungen auch bei hohen Gehalten an Lipiden stabil sein und ein angenehmes Hautgefühl erzeugen.

Überraschend hat sich gezeigt, dass eine
kosmetische Zubereitung in Form einer Hydrodispersion, dadurch gekennzeichnet, dass sie
i. Wasser,
ii. Natriumcarboxymethylstärke,
iii. ein oder mehrere Hydrokolloide,
iv. ein oder mehrere Silikonöle und
v. bis zu 2 Gew.-% eines oder mehrerer Emulgatoren enthält
diese Aufgaben zu lösen vermag.

Besonders bevorzugt sind die erfindungsgemäßen Zubereitungen frei von Emulgatoren.

Die erfindungsgemäßen Zubereitungen stellen eine erhebliche Bereicherung des Standes der Technik in Bezug auf feindisperse Zubereitungen dar. Sie sind in jeglicher Hinsicht überaus befriedigende Präparate. Es war für den Fachmann nicht vorauszusehen gewesen, dass die erfindungsgemäßen Hydrodispersionen insbesondere sehr gehaltvoll und pflegend sein würden, wobei sie ein samtig-seidiges Hautgefühl nach ihrer Anwendung erzeugen: Die Haut fühlt sich angenehm zart und geschmeidig an, wobei diese Wirkung den ganzen Tag anhalten kann.

Als Maß für die Pflegeleistung eines Kosmetikums kann beispielsweise die Wirkung des Produktes auf die Hautfeuchte dienen. Der Wassergehalt der Hornschicht spielt als Schutzfaktor (Barrierefunktion), aber auch beim kosmetischen Eindruck der Haut eine große Rolle. Ist er zu gering, wird die Haut trocken und rissig und kann ihre Barrierefunktion nicht mehr erfüllen. Der Grad der Befeuchtung der Hornschicht lässt sich durch Corneometrie ermitteln. Als Messsonde dient dabei ein Plattenkondensator, an dessen Rändern sich ein elektrisches Streufeld ausbildet.

Die Eigenschaften dieses elektrischen Feldes ändern sich mit dem Wassergehalt der Umgebung, durch die es verläuft. Der Plattenkondensator ist dabei durch eine hauchdünne Folie von der Haut getrennt, die verhindert, dass durch das ionenhaltige Oberflächenwasser Kurzschlüsse zwischen den Kondensatorplatten entstehen. Die Messung erfolgt in einem vollklimatisierten, konstant temperierten Raum, da das Messergebnis stark von der Aktivität der Schweißdrüsen beeinflusst werden kann.

Zur Untersuchung der Wirkung des Produktes wird die Hautfeuchtigkeit jeweils vor und zu einem definierten Zeitpunkt (beispielsweise 2 Stunden) nach einer kontollierten Anwendung ermittelt. Es ist vorteilhaft, die Prüfmuster gegen ein unbehandeltes Kontrollareal zu prüfen.

Ferner war es überraschend, dass sich die erfindungsgemäßen Hydrodispersionen auch bei höheren Lipidgehalten von bis zu 10 Gew.-% durch eine sehr gute Lagerstabilität auszeichnen. So sind derartige Zubereitungen beispielsweise selbst bei einer Temperatur von 40 °C mindestens 3 Monate lagerstabil, d. h. sie zeigen keinerlei Phasentrennung.

Natriumcarboxymethylstärke mit der CAS-Nr. 9063-38-1 trägt die INCI-Bezeichnung Sodium carboxy methyl starch und ist beispielsweise unter der Handelsbezeichnung COVAGEL von der Fa. LCW erhältlich.

Es ist bevorzugt im Sinne der vorliegenden Erfindung, die Menge an Natriumcarboxymethylstärke aus dem Bereich von 0,5 bis 5 Gew.-%, besonders bevorzugt aus dem Bereich von 2 bis 3 Gew.-% - jeweils bezogen auf das Gesamtgewicht der Zubereitung - zu wählen.

Das oder die Hydrokolloide werden erfindungsgemäß vorteilhaft aus einer oder mehreren der folgenden Gruppen gewählt:
- organische, natürliche Verbindungen, wie beispielsweise Agar-Agar, Carrageen, Tragant, Gummi arabicum, Alginate, Pektine, Polyosen, Guar-Mehl, Johannisbrotbaumkernmehl, Stärke, Dextrine, Gelatine, Casein,
- organische, abgewandelte Naturstoffe, wie z. B. Carboxymethylcellulose und andere Celluloseether, Hydroxyethyl- und -propylcellulose und mikrokristalline Cellulose dergleichen,
- organische, vollsynthetische Verbindungen, wie z. B. Polyacryl- und Polymethacryl-Verbindungen, Vinylpolymere, Polycarbonsäuren, Polyether, Polyimine, Polyamide, Polyurethane.

Erfindungsgemäß besonders vorteilhafte Hydrokolloide sind Verbindungen, die die INCI-Bezeichnung Ammonium Acryloyldimethyltaurate / VP Copolymer tragen.

Erfindungsgemäß vorteilhaft weisen diese Verbindungen die Summenformel [C₇H₁₆N₂SO₄]ₙ [C₆H₉NO]ₘ auf, einer statistischen Struktur wie folgt entsprechend

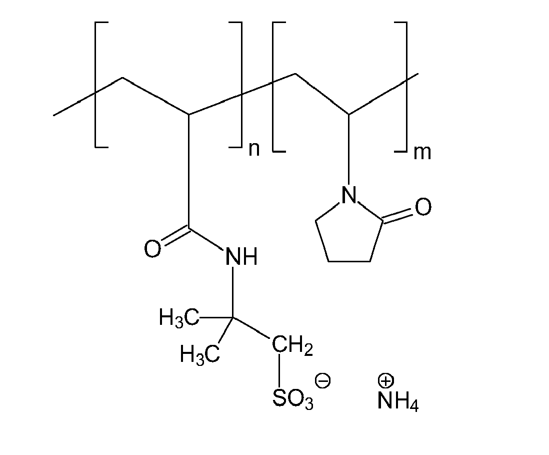

Bevorzugte Spezies im Sinne der vorliegenden Erfindung sind in den Chemical Abstracts unter den Registraturnummern 58374-69-9, 13162-05-5 und 88-12-0 abgelegt und erhältlich unter der Handelsbezeichnung Aristoflex^{®} AVC der Gesellschaft Clariant GmbH.

Im folgenden werden diese Verbindungen auch als Ammoniumacryloyldimethyltaurate/Vinylpyrrolidoncopolymere bezeichnet.

Vorteilhaft sind ferner Copolymere/Crosspolymere umfassend Acryloyldimethyl Taurate, wie beispielsweise Simugel^{®} EG oder Simugel^{®} EG von der Gesellschaft Seppic S.A.

Besonders bevorzugte Hydrokolloide im Sinne der vorliegenden Erfindung sind ferner Polyacrylate, d. h. Polymere auf Basis von Estern der Acrylsäure *(Polyacrylsäureester)* der allgemeinen Strukturformel 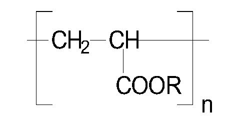 worin R für lineare, verzweigte oder cyclische, ggf. funktionelle Substituenten (z. B. Hydroxy-, Amin- oder Epoxid-Gruppen) enthaltende Alkyl-Reste steht, z. B. für Methyl-, Ethyl-, Isopropyl-, *tert-*Butyl-, Cyclohexyl-, 2-Ethylhexyl-, Dodecyl-, 2-Hydroxyethyl- und 2-Dimethylaminoethyl-.

Ganz besonders bevorzugt sind Polyacrylate mit der INCI-Bezeichnung Sodium Polyacrylate, beispielsweise das unter der Handelsbezeichnung Cosmedia SP bei der Fa. Cognis erhältliche.

Es ist bevorzugt im Sinne der vorliegenden Erfindung, das oder die Hydrokolloide aus der Gruppe Ammoniumacryloyldimethyltaurate/Vinylpyrrolidoncopolymere und Polyacrylate zu wählen.

Das Verhältnis von Natriumcarboxymethylstärke zu Hydrokolloid (insbesondere Ammoniumacryloyldimethyltaurate/Vinylpyrrolidoncopolymere und/oder Sodium Polyacrylate) wird bevorzugt wie 5 : 1 bis 1 : 1 gewählt. Besonders bevorzugt ist ein Verhältnis von ca. 2 : 1.

Die erfindungsgemäßen Zubereitungen weisen einen Gehalt an cyclischen und/oder linearen Silikonölen auf.

Vorteilhaft werden Cyclomethicone, insbesondere Octamethylcyclotetrasiloxan, Cyclomethicone D5 und/oder Cyclomethicone D6 als erfindungsgemäßes Silikonöl eingesetzt. Ein weiteres erfindungsgemäß vorteilhaftes Silikonöl ist Dimethicon (auch: Dimethylpolysiloxan bzw. Polydimethylsiloxan mit der INCI-Bezeichnung Dimethicone).

Aber auch andere Silikonöle sind vorteilhaft im Sinne der vorliegenden Erfindung zu verwenden, beispielsweise Hexamethylcyclotrisiloxan, Poly(methylphenylsiloxan), Phenyltrimethicon, Phenyldimethicon, Hexamethylcyclotrisiloxan, Polydimethylsiloxan, Poly(methylphenylsiloxan), Cetyldimethicon, Behenoxydimethicon.

Es ist aber auch vorteilhaft, Silikonöle ähnlicher Konstitution wie der vorstehend bezeichneten Verbindungen zu wählen, deren organische Seitenketten derivatisiert, beispielsweise polyethoxyliert und/oder polypropoxyliert sind. Dazu zählen beispielsweise Poly-siloxan-polyalkyl-polyethercopolymere wie das Cetyl-Dimethicon-Copolyol oder auch das Lauryl-Methicon-Copolyol.

Besonders vorteilhaft zur Verwirklichung der erfindungsgemäßen Textur wird eine Mischung von cyclischen und linearen Silikonölen, insbesondere von Cyclomethicon und Dimethicon, verwendet. Es ist besonders bevorzugt im Sinne der vorliegenden Erfindung, in diesem Fall das Gewichtsverhältnis von Cyclomethicon zu Dimethicon wie a : b zu wählen, wobei a und b unabhängig voneinander rationale Zahlen von 1 bis 10 darstellen können. Insbesondere bevorzugt ist ein Gewichtsverhältnis von etwa 1 : 1 bis 1 : 10.

Es ist bevorzugt im Sinne der vorliegenden Erfindung, wenn die kosmetischen Hydrodispersionen ein oder mehrere Silikonöle (besonders bevorzugt Cyclomethicon und Dimethicon) in einer Gesamtmenge von 0,5 bis 10 Gew.-%, besonders 1 bis 7 Gew.-%, ganz besonders 3 bis 5 Gew.-% - jeweils bezogen auf das Gesamtgewicht der Zubereitung - enthalten.

In einer besonders bevorzugten Ausführungsform, mit der sich die erfindungsgemäße samtige Textur besonders gut verwirklichen lässt, wobei die Formulierungen selbst besonders gehaltvoll und pflegend sind und gleichzeitig für ein samtweiches Hautgefühl nach der Anwendung sorgen, enthalten die Zubereitungen im Sinne der vorliegenden Erfindung sog. Moisturizer. Als Moisturizer werden Stoffe oder Stoffgemische bezeichnet, welche kosmetischen Zubereitungen die Eigenschaft verleihen, nach dem Auftragen bzw. Verteilen auf der Hautoberfläche die Feuchtigkeitsabgabe der Hornschicht (auch transepidermal water loss (TEWL) genannt) zu reduzieren und/oder die Hydratation der Hornschicht positiv zu beeinflussen.

Vorteilhafte Moisturizer im Sinne der vorliegenden Erfindung sind beispielsweise Glycerin, Milchsäure und/oder Lactate, insbesondere Natriumlactat, Butylenglykol, Propylenglykol, Biosaccaride Gum-1, Glycine Soja, Ethylhexyloxyglycerin, Pyrrolidoncarbonsäure und Harnstoff.

Vorteilhaft wird die Menge an Moisturizern (eine oder mehrere Verbindungen) aus dem Bereich von 1 bis 20 Gew.-%, bevorzugt von 3 bis 15 Gew.-%, besonders bevorzugt von 5 bis 10 Gew.-% - jeweils bezogen auf das Gesamtgewicht der Zubereitung - gewählt.

Die wässrige Phase der erfindungsgemäßen Zubereitungen enthält gegebenenfalls vorteilhaft Alkohole, Diole oder Polyole niedriger C-Zahl, sowie deren Ether, vorzugsweise Ethanol, Isopropanol, 1,2-Propandiol, Propylenglykol, Glycerin, Ethylenglykol, Ethylenglykolmonoethyl- oder -monobutylether, Propylenglykolmonomethyl, -monoethyl- oder -monobutylether, Diethylenglykolmonomethyl- oder -monoethylether und analoge Produkte.

Es ist besonders vorteilhaft im Sinne der vorliegenden Erfindung, wenn die Zubereitungen Ethanol enthalten. Die Menge an Ethanol wird dann bevorzugt aus dem Bereich von 1 bis 10 Gew.-%, bevorzugt von 2,5 bis 7,5 Gew.-%, besonders bevorzugt wie 5 Gew.-% - jeweils bezogen auf das Gesamtgewicht der Zubereitungen - gewählt.

Besonders vorteilhafte Zubereitungen werden ferner erhalten, wenn als Zusatz- oder Wirkstoffe Antioxidantien eingesetzt werden. Erfindungsgemäß enthalten die Zubereitungen vorteilhaft eines oder mehrere Antioxidantien. Als günstige, aber dennoch fakultativ zu verwendende Antioxidantien können alle für kosmetische und/oder dermatologische Anwendungen geeigneten oder gebräuchlichen Antioxidantien verwendet werden.

Die Menge der Antioxidantien (eine oder mehrere Verbindungen) in den Zubereitungen beträgt vorzugsweise 0,001 bis 30 Gew.-%, besonders bevorzugt 0,05 bis 20 Gew.-%, insbesondere 1 bis 10 Gew.-%, jeweils bezogen auf das Gesamtgewicht der Zubereitung.

Besonders vorteilhaft im Sinne der vorliegenden Erfindung können öllösliche Antioxidantien eingesetzt werden. Bevorzugte Antioxidantien sind Vitamin E und dessen Derivate sowie Vitamin A und dessen Derivate.

Sofern Vitamin E und/oder dessen Derivate das oder die Antioxidantien darstellen, ist vorteilhaft, deren jeweilige Konzentrationen aus dem Bereich von 0,001 bis 10 Gew.-%, bezogen auf das Gesamtgewicht der Formulierung, zu wählen.

Sofern Vitamin A, bzw. Vitamin-A-Derivate, bzw. Carotine bzw. deren Derivate das oder die Antioxidantien darstellen, ist vorteilhaft, deren jeweilige Konzentrationen aus dem Bereich von 0,001 bis 10 Gew.-%, bezogen auf das Gesamtgewicht der Formulierung, zu wählen.

Es ist dem Fachmann natürlich bekannt, dass kosmetische Zubereitungen zumeist nicht ohne die üblichen Hilfs- und Zusatzstoffe denkbar sind. Die erfindungsgemäßen kosmetischen Zubereitungen können dementsprechend - je nach gewünschtem Anwendungszweck - ferner kosmetische Hilfsstoffe enthalten, wie sie üblicherweise in solchen Zubereitungen verwendet werden, beispielsweise Konsistenzgeber, Stabilisatoren, Füllstoffe, Konservierungsmittel, Parfüme, Substanzen zum Verhindern des Schäumens, Farbstoffe, Pigmente, die färbende Wirkung haben, oberflächenaktive Substanzen, Emulgatoren, weichmachende, anfeuchtende und/oder feuchthaltende Substanzen, entzündungshemmende Substanzen, zusätzliche Wirkstoffe wie Vitamine oder Proteine, Lichtschutzmittel, Insektenrepellentien, Bakterizide, Viruzide, Salze, antimikrobiell, proteolytisch oder keratolytisch wirksame Substanzen oder andere übliche Bestandteile einer kosmetischen Formulierung wie Polyole, Polymere, Schaumstabilisatoren, organische Lösungsmittel oder auch Elektrolyte.

Letztere können beispielsweise gewählt werden aus der Gruppe der Salze mit folgenden Anionen: Chloride, ferner anorganische Oxo-Element-Anionen. Auch auf organischen Anionen basierende Elektrolyte sind vorteilhaft, z. B. Lactate, Acetate, Benzoate, Citrate, Aminosäuren, Ethylendiamintetraessigsäure und deren Salze und andere mehr. Als Kationen der Salze werden bevorzugt Ammonium,- Alkalimetall-, Erdalkalimetall,- Magnesium-, Eisen- bzw. Zinkionen verwendet. Es bedarf an sich keiner Erwähnung, dass in Kosmetika nur physiologisch unbedenkliche Elektrolyte verwendet werden sollten. Besonders bevorzugt sind Kochsalz, Magnesiumsulfat, Magnesiumchlorid, Zinksulfat und Mischungen daraus.

Vorteilhaft enthalten die Zubereitungen im Sinne der vorliegenden Erfindung ferner einen oder mehrere Wirkstoffe gewählt aus der Gruppe: Ceramid 3, Traubenkernöl, Biotin, Panthenol, Aloe Vera, Hammamelis-Extrakt, Ginkgo-Extrakt, Honig, Weizenkeimöl und Mandelöl.

Die erfindungsgemäßen Hydrodispersionen können als Grundlage für kosmetische Formulierungen dienen. Diese können wie üblich zusammengesetzt sein und beispielsweise zur Behandlung und der Pflege der Haut und/oder der Haare, als Lippenpflegeprodukt, als Deoprodukt und als Schmink- bzw. Abschminkprodukt in der dekorativen Kosmetik oder als Lichtschutzpräparat dienen. Zur Anwendung werden die erfindungsgemäßen kosmetischen und dermatologischen Zubereitungen in der für Kosmetika oder Dermatika üblichen Weise auf die Haut und/oder die Haare in ausreichender Menge aufgebracht.

Entsprechend können kosmetische Zusammensetzungen im Sinne der vorliegenden Erfindung, je nach ihrem Aufbau, beispielsweise verwendet werden als Hautschutzcrème, Reinigungsmilch, Sonnenschutzlotion, Nährcrème, Tages- oder Nachtcrème usw. Besonders bevorzugt im Sinne der vorliegenden Erfindung sind Zubereitungen zur (großflächigen) Anwendung im Körperpflegebereich.

Günstig im Sinne der vorliegenden Erfindung sind auch Zubereitungen, die in der Form eines Sonnenschutzmittels vorliegen. Es ist aber auch vorteilhaft im Sinne der vorliegenden Erfindungen, solche Zubereitungen zu erstellen, deren hauptsächlicher Zweck nicht der Schutz vor Sonnenlicht ist, die aber dennoch einen Gehalt an UV-Schutzsubstanzen enthalten. So werden z. B. in Tagescrèmes gewöhnlich UV-A- bzw. UV-B-Filtersubstanzen eingearbeitet. Vorzugsweise enthalten solche Zubereitungen im Sinne der vorliegenden Erfindung mindestens eine UV-A-Filtersubstanz und/oder mindestens eine UV-B-Filtersubstanz und/oder mindestens ein anorganisches Pigment, zumal UV-Schutzsubstanzen, ebenso wie Antioxidantien und - gewünschtenfalls - Konservierungsstoffe, einen wirksamen Schutz der Zubereitungen selbst gegen Verderb darstellen.

Es ist vorteilhaft im Sinne der vorliegenden Erfindung, wenn die verwendeten UV-Filtersubstanzen wasserlöslich sind.

Erfindungsgemäß vorteilhafte wasserlösliche UV-Filtersubstanzen sind z. B.:
■ Salze der 2-Phenylbenzimidazol-5-sulfonsäure, wie ihr Natrium-, Kalium- oder ihr Triethanolammonium-Salz, sowie die Sulfonsäure selbst;
■ Sulfonsäure-Derivate des 3-Benzylidencamphers, wie z. B. 4-(2-Oxo-3-bornylidenmethyl)benzolsulfonsäure, 2-Methyl-5-(2-oxo-3-bornylidenmethyl)sulfonsäure und deren Salze.

Ferner vorteilhaft im Sinne der vorliegenden Erfindung sind an Polymere gebundene öllösliche UV-Filter, wie z. B. Polysilicone-15, das auch unter dem Handelsnamen Parsol SLX erhältlich ist.

Es ist bevorzugt im Sinne der vorliegenden Erfindung Polysilicone-15 und 2-Phenylbenzimidazol-5-sulfonsäure zu kombinieren.

Die Gesamtmenge der Filtersubstanzen wird aus dem Bereich von 0,1 bis 30 Gew.-%, vorzugsweise 0,5 bis 10 Gew.-%, insbesondere 1,0 bis 6,0 Gew.-% - jeweils bezogen auf das Gesamtgewicht der Zubereitungen - gewählt, um kosmetische Zubereitungen zur Verfügung zu stellen, die das Haar bzw. die Haut vor dem gesamten Bereich der ultravioletten Strahlung schützen.

Die nachfolgenden Beispiele sollen die vorliegende Erfindung verdeutlichen, ohne sie einzuschränken. Die Zahlenwerte in den Beispielen bedeuten Gewichtsprozente, bezogen auf das Gesamtgewicht der jeweiligen Zubereitungen.

### Beispiele:

| Beispiel | 1 | 2 | 3 | 4 | 5 | 6 | 7 | 8 |
|---|---|---|---|---|---|---|---|---|
| Glyceryl Stearate Citrat | | 1,0 | | | | | | |
| Stearinsäure / Palmitinsäure | 0,5 | | | | | | | |
| PEG-100 Stearat | 1,0 | | | | | | | |
| Cetyl Alkohol | | 0,5 | | | | | | |
| Stearyl Alcohol | | 0,5 | | | | | | |
| Cetearyl Alkohol | 0,5 | | | | | | | |
| Caprylic/Capric Triglycerid | | | | | | | | 0,5 |
| Ethylhexylcocoat | | | | | | | 1,0 | |
| Octyldodecanol | | 1,0 | | | | | | |
| Mineral Oil | | | 2,0 | | | | | |
| Hydriertes Polyisobuten | 1,0 | | | | | | | |
| Polydecen | | | | 1,0 | | | | |
| Cyclomethicon | 5,0 | 2,0 | 5,0 | 1,0 | | 7,0 | 1,0 | |
| Dimethicon | 5,0 | 3,0 | | 6,0 | 10,0 | | 3,0 | 7,0 |
| Phenyltrimethicon | | | | | | 1,0 | | |
| Dicarprylyl Carbonat | | | | | 1,0 | | | |
| Natürliche Öle (z.B. Triglyceride wie Jojobaöl) | 0,5 | | | 0,25 | | | | |
| Natriumcarboxymethylstärke | 0,5 | 5,0 | 2,0 | 3,0 | 2,5 | 4,0 | 1,0 | 2,5 |
| Natrium Polyacrylate | 0,25 | 0,1 | 0,5 | | 1,25 | | 0,25 | 0,5 |
| Ammoniumacryloyldimethyltaurat / Vinypyrrolidoncopolymer | 0,25 | 0,2 | 0,5 | 1,5 | | 2,0 | 0,25 | 0,5 |
| Trisodium EDTA | 0,2 | 0,1 | | 0,05 | | 0,1 | 0,3 | |
| Iminodisuccinat | 0,1 | | 0,1 | | 0,3 | | | 0,5 |
| Phenoxyethanol | 0,3 | 0,1 | | 0,5 | 0,8 | | | 0,4 |
| Parabene | 0,6 | | 0,4 | | 0,4 | | 0,2 | |
| Hexamidin Diisethionat | | 0,1 | | | 0,05 | | 0,1 | |
| Imidodiazolydynl Urea | | | | | | 0,2 | | 0,2 |
| DMDM Hydantoin | | | 0,2 | | | 0,1 | | |
| lodopropynyl Butylcarbamat | | | | 0,2 | | | 0,05 | |
| Glycerin | 10,0 | 3,0 | 7,0 | 8,0 | 15,0 | 20,0 | 0,5 | 2,0 |
| Harnstoff | | 2,0 | | | | | 5,0 | 2,0 |
| Alcohol Denat. | 5,0 | | 2,5 | | | 7,5 | | 7,5 |
| Wasser | ad 100 | ad 100 | ad 100 | ad 100 | ad 100 | ad 100 | ad 100 | ad 100 |

## Patentansprüche

1. Kosmetische Zubereitung in Form einer Hydrodispersion, **dadurch gekennzeichnet, dass** sie
i. Wasser,
ii. Natriumcarboxymethylstärke,
iii. ein oder mehrere Hydrokolloide,
iv. ein oder mehrere Silikonöle und
v. bis zu 2 Gew.-% eines oder mehrerer Emulgatoren enthält.

2. Zubereitung nach Anspruch 1, **dadurch gekennzeichnet, dass** die Menge an Natriumcarboxymethylstärke aus dem Bereich von 0,5 bis 5 Gew.-% - bezogen auf das Gesamtgewicht der Zubereitung - gewählt wird.

3. Zubereitung nach Anspruch 1, **dadurch gekennzeichnet, dass** die Menge an Natriumcarboxymethylstärke aus dem Bereich von 2 bis 3 Gew.-% - bezogen auf das Gesamtgewicht der Zubereitung - gewählt wird.

4. Zubereitung nach einem der vorhergehenden Ansprüche, **dadurch gekennzeichnet, dass** als Hydrokolloid ein Ammoniumacryloyldimethyltaurate/Vinylpyrrolidoncopolymer gewählt wird.

5. Zubereitung nach einem der vorhergehenden Ansprüche, **dadurch gekennzeichnet, dass** als Hydrokolloid ein Sodium Polyacrylat gewählt wird.

6. Zubereitung nach einem der vorhergehenden Ansprüche, **dadurch gekennzeichnet, dass** das Verhältnis von Natriumcarboxymethylstärke zu Hydrokolloid (insbesondere Ammoniumacryloyldimethyltaurate/Vinylpyrrolidoncopolymere und/oder Sodium Polyacrylate) wie 5 : 1 bis 1 : 1 gewählt wird.

7. Zubereitung nach Anspruch 6, **dadurch gekennzeichnet, dass** das Verhältnis wie 2 : 1 gewählt wird.

8. Zubereitung nach einem der vorhergehenden Ansprüche, **dadurch gekennzeichnet, dass** als Silikonöl Cyclomethicon und/oder Dimethicon gewählt werden.

9. Zubereitung nach einem der vorhergehenden Ansprüche, **dadurch gekennzeichnet, dass** die Menge an dem oder den Silikonölen aus dem Bereich von 0,5 bis 10 Gew.-% - bezogen auf das Gesamtgewicht der Zubereitung - gewählt wird.

10. Zubereitung nach einem der vorhergehenden Ansprüche, **dadurch gekennzeichnet, dass** die Menge an dem oder den Silikonölen aus dem Bereich von 1 bis 7 Gew.-% - bezogen auf das Gesamtgewicht der Zubereitung - gewählt wird.

11. Zubereitung nach einem der vorhergehenden Ansprüche, **dadurch gekennzeichnet, dass** sie ferner einen oder mehrere Moisturizer enthält.

12. Zubereitung nach Anspruch 11, **dadurch gekennzeichnet, dass** das die Menge an dem oder den Moisturizern aus dem Bereich von 1 bis 20 Gew.-% - bezogen auf das Gesamtgewicht der Zubereitung - gewählt wird.

13. Zubereitung nach Anspruch 11, **dadurch gekennzeichnet, dass** das die Menge an dem oder den Moisturizern aus dem Bereich von 3 bis 15 Gew.-% - bezogen auf das Gesamtgewicht der Zubereitung - gewählt wird.

14. Zubereitung nach Anspruch 11, **dadurch gekennzeichnet, dass** der oder die Moisturizer aus der Gruppe Glycerin, Milchsäure und/oder Lactate, insbesondere Natriumlactat, Butylenglykol, Propylenglykol, Biosaccaride Gum-1, Glycine Soja, Ethylhexyloxyglycerin, Pyrrolidoncarbonsäure und Harnstoff gewählt wird/werden.

15. Zubereitung nach einem der vorhergehenden Ansprüche, **dadurch gekennzeichnet, dass** sie ferner Ethanol enthält.

16. Zubereitung nach Anspruch 15, **dadurch gekennzeichnet, dass** das die Menge an Ethanol aus dem Bereich von 1 bis 10 Gew.-% - bezogen auf das Gesamtgewicht der Zubereitung - gewählt wird.

17. Zubereitung nach Anspruch 15, **dadurch gekennzeichnet, dass** das die Menge an Ethanol aus dem Bereich von 2,5 bis 7,5 Gew.-% - bezogen auf das Gesamtgewicht der Zubereitunggewählt wird.
